# EUROPEAN PATENT SPECIFICATION

(11) **EP 1 330 282 B1**
(45) Date of publication and mention of the grant of the patent: **07.12.2005**
(21) Application number: 01992592.4
(22) Date of filing: 19.10.2001
(51) Int. Cl.: A61M 15/00

(54) **MEDICAMENT DISPENSER**
SPENDER FÜR MEDIKAMENT
DISTRIBUTEUR DE MEDICAMENT

(30) Priority: 31.10.2000 GB 0026647; 08.12.2000 WO PCT/EP00/12389
(43) Date of publication of application: 30.07.2003
(73) Proprietor: GLAXO GROUP LIMITED, Greenford, Middlesex UB6 ONN (GB)
(72) Inventor: RAND, Paul, Kenneth, Glaxo Wellcome plc, Ware, Herts SG12 0DP (GB); HARVEY, Stephen, James, Glaxo Wellcome plc., Ware, Herts SG12 ODP (GB)
(74) Representative: Rice, Jason Neale
(86) International application number: PCT/EP2001/012106
(87) International publication number: WO 2002/036188

(56) References cited:
- EP-A- 0 938 907
- US-A- 5 829 435
- US-A- 5 873 360

## Description

The present invention relates to a medicament dispenser for dispensing medicament. The invention particularly relates to a device for use in dispensing medicament in powder or tablet form.

### Background to the invention

The use of inhalation devices in the administration of medicaments, for example in bronchodilation therapy is well known. Such devices generally comprise a body or housing within which a medicament carrier is located. Known inhalation devices include those in which the medicament carrier is a blister strip containing a number of discrete doses of powdered medicament. Such devices usually contain a mechanism of accessing these doses, usually comprising either piercing means or means to peel a lid sheet away from a base sheet. The powdered medicament can then be accessed and inhaled. Such a mechanism may also be used for dispensing medicament in tablet form wherein peeling away the lid sheet from the base sheet reveals a tablet for removal and subsequent consumption.

It is an object of the present invention to simplify the internal mechanism of a medicament dispenser for dispensing medicament in powder or solid form from a medicament carrier as described *supra.*

Yet another object of the present invention is to provide a device which is refillable by insertion of a replacement cassette containing a medicament carrier. The cassette may be replaced when the medicament carrier is empty. The device is therefore more 'environmentally friendly' as the majority of the device may be retained and is not disposable. It also allows the device to be fitted with additional features such as electronics which may not be cost effective on a completely disposable device.

It is a further object of the present invention that the cassette may be easily removed and that a new replacement cassette can be easily inserted. It is also desirable that the operation of the medicament dispenser be straightforward and non-comple and in particular that the number of separate steps involved in preparing the device for use be minimised. This is especially relevant where the device is designed for use in the delivery of medicament in emergency or rescue situations (e.g. asthma attacks) where simplicity and ease of use is paramount.

When not in use it is desirable from a hygiene standpoint that a mouthpiece, or other medicament exit channel, is provided with some kind of protective cover. The cover desirably acts both to prevent build-up of dirt and to prevent ingress of dirt into the body of the device through the mouthpiece or channel, which might then be subject to inhalation or consumption by a patient. It is also desirable that the cover is in some way attached or mounted to the device to minimise the risk that the cover is misplaced or lost. It is therefore a further object of the present invention for the body of the device to act as a mouthpiece or exit channel cover when the device is in storage and that the cassette is movable relative to the body to enable the mouthpiece or channel to be uncovered for use by the patient.

It is a further object of the invention to provide a medicament dispenser device suitable for use with a large number of discrete doses but which is of an acceptable size for use by patients.

US-A-5 873 360 discloses a medicament dispenser according to the pre-characterising part of claim 1 hereof. Background art is also disclosed in US-A-5 829 435.

### Summary of the invention

Accordingly, in one aspect the invention provides a medicament dispenser as set out in claim 1.

The electronic drive system may also be used in conjunction with a mechanical drive system.

The drive means typically comprises a motor, preferably an electrically-powered motor. The motor may provide linear or rotary drive, but in general, rotary motors are most suitable. The motor may for example, comprise a DC electric motor, a piezoelectric (PZ) motor, an ultrasonic motor, a solenoid motor or a linear motor. Preferably, the electronic drive system comprises a DC motor, a PZ motor or an ultrasonic motor.

The use of ultrasonic motors is particularly preferred since they offer advantages over conventional motors in terms of weight, size, noise, cost and torque generated. Ultrasonic motors are well known in the art and are commercially available (e.g. BMSTU Technological Cooperation Centre Ltd, Moscow, Russia; Shinsei Corporation, Tokyo, Japan).

Ultrasonic motors do not use coils or magnets but comprise a piezo-electric ceramic stator which drives a coupled rotor. The stator generates ultrasonic vibrations which in turn causes rotation of the rotor. While regular DC motors are characterised by high speed and low torque, requiring reduction gearing to increase torque, ultrasonic motors attain low speed and high torque, thus eliminating the need for reduction gearing. Furthermore, these motors are lightweight and compact, lacking coils and magnets, and are noiseless as the ultrasonic frequencies used are not audible to the human ear.

Suitably, the dispenser further comprises actuating means for actuating said electronic drive system. Said actuating means may take the form of a switch, push-button, or lever.

Suitably, said indexing means comprises a rotatable index wheel having a recesses therein, said index wheel being engageable with a medicament carrier in use with said medicament dispenser such that said recesses each receive a respective pocket of the base sheet of a medicament carrier in use with said medicament dispenser.

Suitably, said rotatable index wheel additionally comprises a series of indentations located at its base and spaced in between the recesses.

Suitably, the indexing means additionally comprises an interlock coupling to couple actuation of the dispenser to the index wheel. The interlock coupling reversibly locks the index wheel in place. Preferably, said interlock coupling comprises a foot portion having a toe and a heel, and a tail section. Preferably, said interlock coupling is pivotally mountable to the dispenser at its foot portion. Preferably, said toe fits into one of the indentations on the rotatable index wheel. Preferably, the interlock coupling is sprung to bias it towards location of the toe in one of the indentations.

Alternatively, said indexing means comprises a gear and sprocket wherein teeth on the wheel fit into apertures or holes formed on one or both edges of the medicament, carrier (e.g. blister strip). The mechanism therefore resembles that of photographic film being advanced through a camera.

Alternatively, said indexing means comprises an index ratchet which is moveable between a locked position whereby said ratchet engages a pocket on said medicament carrier and prevents further peeling thereof, and a release position allowing free movement of said medicament carrier. In this embodiment, actuation of said medicament dispenser actuates said lid driving means and releases said index ratchet from said medicament carrier to allow peeling thereof.

Suitably, said peeling means additionally comprise a guide for guiding the lid sheet and base sheet along separate paths at the opening station. The lid sheet is passed around the guide portion onto the lid driving means.

Alternatively, the guide comprises a roller mechanism. The lid sheet is fed over the rollers onto the lid driving means.

Suitably, the internal mechanism additionally comprises a first chamber in which the strip is initially housed and from which it is dispensed and a second chamber to receive the used portion of the base sheet after it has been indexed around the index wheel and separated from the lid sheet.

Suitably, said first chamber and said second chamber are separated by a wall.

Suitably, said wall is movable to adjust the size of said first and second chambers.

In one aspect, the wall is pivotally mountable. Alternatively the wall is slidably mountable.

Typically, the internal mechanism for accessing said medicament contained within said medicament carrier is housed within a cassette.

Thus, in another embodiment, the invention provides a medicament dispenser for dispensing medicament comprising: a body; a holder, shaped to fit within said body and movable relative to said body; and receivable by said holder, said cassette containing the medicament carrier.

Suitably, the electronic drive system is located in either the body or the holder part, and the cassette comprises the minimum number of component (i.e. internal mechanism) parts. In embodiments, the body/holder including the electronic drive is designed to be retained by the user and the cassette is sold as a refill/reload component which is discarded after use. By locating the electronic drive system in the body/holder, the amount of electronic components which are discarded is minimised which is advantageous from an environmental standpoint

Suitably, the cassette of the medicament dispenser herein comprises
a) an opening station for receiving a pocket of the medicament carrier,
b) peeling means positioned to engage a base sheet and a lid sheet of a pocket which has been received in said opening station for peeling apart such a base sheet and lid sheet, to open such a pocket, said peeling means including lid driving means for pulling apart a lid sheet and a base sheet of a pocket that has been received at said opening station;
c) an outlet, positioned to be in communication with an opened pocket through which a user can remove medicament from such an opened pocket; and
d) indexing means for indexing in communication with said outlet, pockets of a medicament carrier in use with said medicament dispenser,
wherein the lid driving means and/or said indexing means are operable by an electronic drive system located in the body or holder part.

Suitably, movement of the holder relative to the body results in movement of the cassette between a first position and a second position such that the cassette is reversibly removable from the holder when the cassette is in the second position.

Suitably the first position comprises a dispensing position. Preferably the second position comprises a non-dispensing position. The cassette is therefore only removable from the holder when the cassette is in the non-dispensing position.

Suitably, the holder and body include attaching means to attach the holder to the body. Preferably, said attaching means comprise a snap fit mechanism. Suitably said snap fit mechanism comprises a pin and hole system.

Suitably, the holder is pivotally movable relative to the body. Alternatively the holder is rotationally movable relative to the body.

Suitably the holder additionally comprises a stop to limit movement of the holder relative to the body. The stop abuts against the edge of the body at two points when it is rotated. At these points the holder may be designed to click into place. Therefore when the stop abuts one body edge then it is clicked into the dispensing position and when the stop abuts the other body edge then it is clicked into the non-dispensing position.

Alternatively the holder is slidably movable relative to the body.

Suitably, the holder additionally comprises a catch to retain the cassette. The catch may for example comprise a sprung pin which fits into a hole or an integral catch which deforms when pressed allowing removal of the cassette.

Suitably, the catch is child resistant Child resistance may be realised by having a system which forces the user to perform two actions at once to remove the cassette. Other features of the catch may include shock or impact resistance, the ability to lock the catch and orientation features to ensure that the cassette can only be inserted one way. The catch should also be easy to manufacture and assemble, be robust, be composed of a minimal number of components and intrude minimally into the space into which the cassette is inserted.

Suitably, the holder includes guide means to guide the cassette into the holder. Preferably said guide means comprise guide rails. Alternatively the guide means comprise grooves, indentations or other shaping or surface details to define a 'lock and key' relationship between the holder and the cassette. Colour guides, arrows and any other surface markings may also be employed.

Suitably, the cassette additionally comprises means to actuate the dispenser. The actuating means may take the form of a switch, push-button or lever.

Suitably, the cassette additionally comprises a mouthpiece.

Suitably, said mouthpiece is extendable. The mouthpiece extends as the cassette and holder are moved from the non-dispensing position to the dispensing position.

Alternatively the mouthpiece is retractable. The mouthpiece retracts as the cassette and holder are moved from the dispensing position to the non-dispensing position.

In one aspect, the mouthpiece is telescopic. In another aspect, the mouthpiece is fixed.

The medicament dispenser may also be designed for nasal inhalation of a powdered medicament and may therefore incorporate a nosepiece as an alternative to a mouthpiece. If the medicament is in solid form, the dispenser may incorporate an exit channel for tablet release.

Suitably, the body covers the mouthpiece and indexing lever when the cassette is in the non-dispensing position. This avoids the need for a separate cover and protects the mouthpiece from the ingress of dirt and contaminants during storage.

Suitably, the cassette additionally comprises a raised portion to fit against the holder. The raised portion is located at the opposite end of the cassette to the mouthpiece/nosepiece/exit and indexing lever and prevents the incorrect insertion of the cassette into the holder since it is too wide to fit into the holder. The raised portion is shaped such that it fits against a cut away part of the holder. Preferably said raised portion includes a section which is raised to define a grip portion.

Suitably, at least a portion of the holder and body are shaped for ease of grip by the user.

Suitably, operation of the dispenser may be performed with one hand.

Suitably, the medicament dispenser comprises an actuation or dose counter for counting the number of actuations of the indexing lever or releases of dose from the cassette.

The dose counter may count the number of doses left to be taken or the number of doses taken.

In one aspect, said dose counter is electronic. Alternatively said dose counter is mechanical.

In one aspect, said dose counter is located within the cassette. Alternatively, the dose counter is external to the cassette.

In one aspect, the blister strip has printed numbers on it corresponding to the doses in the pockets. Preferably, said printed numbers are visible through a window in the cassette.

The dispenser may be assembled as follows. The holder is snap fitted into the body. The cassette is assembled separately. The body of the cassette is formed, preferably in two sections with any necessary spindles or integral components formed into the base. Individual components such as indexing wheels, lid winding mechanisms, guide portions etc are then assembled into the base. Finally the medicament containing blister strip (or other suitable medicament carrier) may be inserted into the cassette. This may be wound into the dispenser before the lid is attached to the cassette and the cassette sealed. Alternatively, the cassette may be formed completely apart from a hole left in its side for insertion of the blister strip or medicament carrier. The hole may then be sealed to complete the cassette. This second method of inserting the medicament carrier into the dispenser has the advantage that it is much simpler.

Suitably, the medicament dispenser additionally comprises an electronic data management system. The electronic data management system has input/output capability and comprises a memory for storage of data; a microprocessor for performing operations on said data; and a transmitter for transmitting a signal relating to the data or the outcome of an operation on the data.

Suitably, the electronic data management system is arranged to be responsive to or activated by the voice of a user. Thus, for example the system may be switched on or off in response to a voice command.

The electronic data management system may be integral with the body. Alternatively, the electronic data management system forms part of a base unit which is reversibly associable with the body.

Suitably, the medicament dispenser additionally comprises a data input system for user input of data to the electronic data management system. Preferably, the data input system comprises a man machine interface (MMI) preferably selected from a keypad, voice recognition interface, graphical user interface (GUI) or biometrics interface.

Energy may be conserved by a variety of means to enable the device to operate for longer on a given source of energy, such as a battery. Energy conservation or saving methods have additional advantages in terms of reducing the size requirements of the power source (e.g. battery) and thus the weight and portability of the medicament dispenser.

Suitably, the system additionally comprises a visual display unit for display of data from the electronic data management system to the user. The display may for example, comprise a screen such as an LED or LCD screen. More preferably the visual display unit is associable with the body of the medicament dispenser.

Suitably, the medicament dispenser additionally comprises a datalink for linking to a local data store to enable communication of data between the local data store and the electronic data management system. The datastore may also comprise data management, data analysis and data communication capability,

The datastore may itself form part of a portable device (e.g. a handheld device) or it may be sized and shaped to be accommodated within the patient's home. The datastore may also comprise a physical storage area for storage of replacement cassettes. The datastore may further comprise a system for refilling medicament from a reservoir of medicament product stored therewithin. The datastore may further comprise an electrical recharging system for recharging any electrical energy store on the medicament dispenser, particularly a battery recharging system.

Suitably, the medicament dispenser additionally comprises an actuation detector for detecting actuation of the dispensing mechanism wherein said actuation detector transmits actuation data to the electronic data management system.

The medicament dispenser may additionally comprise a safety mechanism to prevent unintended multiple actuations of the dispensing mechanism. The patient is thereby protected from inadvertently receiving multiple doses of medicament in a situation where they take a number of short rapid breaths. More preferably, the safety mechanism imposes a time delay between successive actuations of the release means. The time delay is typically of the order of from three to thirty seconds.

Suitably, the medicament dispenser additionally comprises a release detector for detecting release of medicament from the cassette, wherein said release detector transmits release data to the electronic data management system.

Suitably, the medicament dispenser additionally comprises a shake detector for detecting shaking of the medicament container (e.g. prior to actuation of the dispensing mechanism), wherein said shake detector transmits shake data to the electronic data management system.

Suitably, the medicament dispenser additionally comprises a breath trigger for triggering the dispensing mechanism, said breath trigger being actuable in response to a trigger signal from the electronic data management system.

A suitable power source such as a battery, clockwork energy store, solar cell, fuel cell or kinetics-driven cell will be provided as required to any electronic component herein. The power source may be arranged to be rechargeable or reloadable.

Preferably the powdered medicament comprises a drug. Preferably the drug is selected from the group consisting of albuterol, salmeterol, fluticasone propionate and beclomethasone dipropionate and salts or solvates thereof and any combination thereof. Preferably said combination comprises salmeterol xinafoate and fluticasone propionate.

Preferably the powdered medicament additionally comprises an excipient. Preferably said excipient is a sugar.

The medicament dispenser may be in the form of a kit of parts.

### Brief Description of the Drawings

The invention will now be described with reference to the accompanying drawings in which:
Figure 1 shows a perspective view of a medicament carrier in accordance with the present invention;
Figure 2 shows a base unit housing an internal mechanism in accordance with one aspect of the invention;
Figure 3 shows a base unit housing an internal mechanism according to another aspect of the invention;
Figure 4a shows a perspective view of a medicament dispenser, in the form of a holder/body and a refill cassette, according to the invention with the cassette removed from the holder/body;
Figures 4b and 4c show side views of the inner workings of the holder of the device of Figure 4a;
Figures 4d and 4e show side views of the inner workings of the cassette of the device of Figure 4a; and
Figure 5 shows a block diagram of an electronic drive system herein.

### Detailed Description of the Drawings

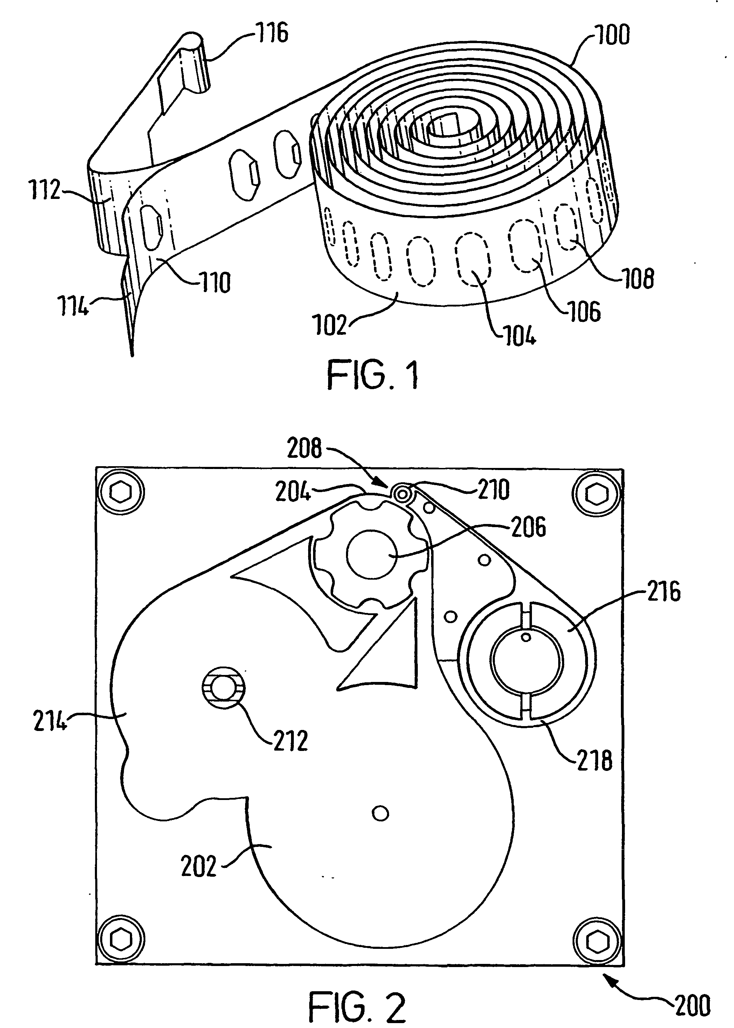

Referring now to the Figures, Figure 1 shows a medicament carrier 100 in accord with the present invention. The medicament carrier comprises a flexible strip 102 defining a plurality of pockets 104, 106, 108 each of which contains a dose of medicament which can be inhaled, in the form of powder.

The strip comprises a base sheet 110 in which blisters are formed to define the pockets 104, 106, 108 and a lid sheet 112 which is hermetically sealed to the base sheet except in the region of the blisters in such a manner that the lid sheet 112 and the base sheet 110 can be peeled apart. The sheets 110, 112 are sealed to one another over their whole width except for the leading end portions 114, 116 where they are preferably not sealed to one another at all. The lid 112 and base 110 sheets are each preferably formed of a plastics/aluminium laminate and are preferably adhered to one another by heat sealing.

The strip 102 is shown as having elongate pockets 104, 106, 108 which run transversely with respect to the length of the strip 102. This is convenient in that it enables a large number of pockets 104, 106, 108 to be provided in a given strip 102 length. The strip 102 may, for example, be provided with sixty or one hundred pockets but it will be understood that the strip 102 may have any suitable number of pockets.

Figure 2 illustrates a base unit 200 of a medicament dispenser according to the invention. A medicament strip (not shown for clarity) is positioned in chamber 202 of the base unit 200. The strip is pre-fed through a guide member 204 within the manifold component and engaged in a six-pocket index wheel 206. The first pocket of the strip is positioned one pocket away from the opening station 208. The lid foil and base foil are separable about a beak 210. The resulting empty base foil is coiled about a base take-up spindle 212 in the base take-up chamber 214. The used lid foil is fed over the beak 210 and coiled about a lid take-up spindle 216 in the lid take-up chamber 218.

The dispenser is actuated by pressing a button on the side of the dispenser (not shown) which is linked to an electronic control system including a DC motor (not shown for clarity) to index the internal mechanism by one pocket of medicament. The DC motor thus indexes the strip and coils up the waste foils.

Initially, the gearing between the index wheel 206 and the lid take-up foil spindle 216 is one-to-one. However, as the lid take up spindle 216 winds on more foil, its effective winding diameter increases. An increase in diameter would cause the lid take-up spindle 216 to pull more strip than the index wheel 206 releases. Thus, in this particular example, the electronic control system is programmed to compensate for increase in lid spool diameter and adjust the amount the lid spool rotates accordingly.

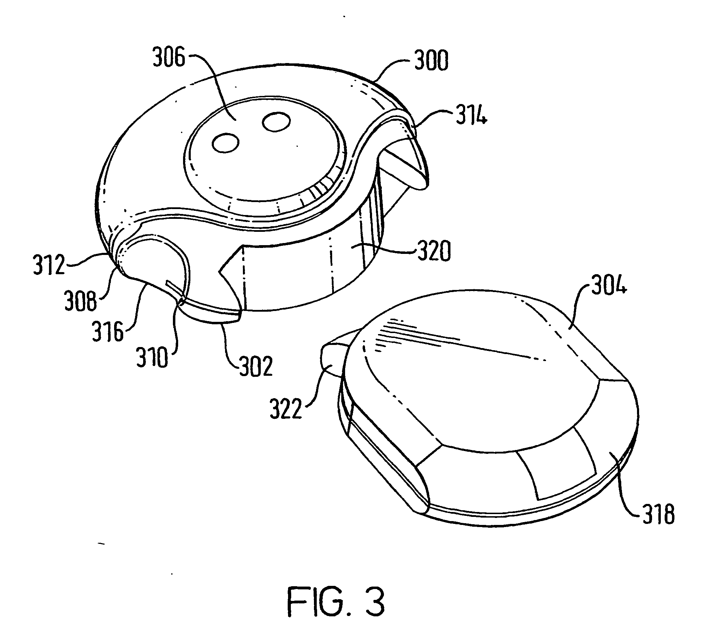

Figure 3 shows a medicament dispenser in accord with the present invention, comprising a body 300, a holder 302, refill cassette 304 and electronic display 306. The holder 302 is shaped to fit snugly inside body 300 and is fixed to a point on the body (not shown) about which it rotates. Stops 308, 310 protrude from the holder 302 and prevent the holder 302 from rotating more than about 180° relative to the body 300. The stops 308, 310 also provide two defined positions of the holder 302 within the body 300. One position is defined by stop 308 meeting with body edge 312 and the other position defined by stop 310 meeting with body edge 314 when the holder has been rotated relative to the body. The area between stops 308 and 310 is shaped to form a thumb or finger grip 316 for the user of the device. The holder 302 forms a shell into which the refill cassette 304 snugly fits.

The refill cassette 304 comprises a shell containing the medicament carrier (not shown) and a mechanism for opening the carrier (not shown) for the medicament to be accessed. The refill cassette 304 has a raised portion 318 at one end on both sides along its width so that this part of the refill cassette 304 is at least the same depth as the part of the holder 320 which receives the refill cassette 304. This allows the position of the cassette 304 within the holder 302 to be fixed such that the ridge 318 protrudes from the holder 302 but the rest of the cassette 304 is contained within the holder 302.

The refill cassette 304 also has a mouthpiece (not shown) and an actuating push button 322 for actuating the DC motor for indexing the medicament carrier within the cassette 304.

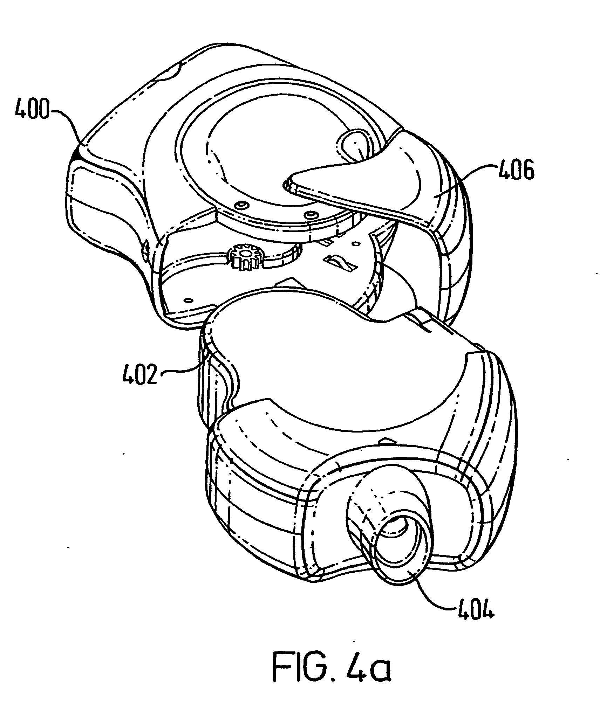

Figures 4a to 4e illustrate a medicament dispenser according to another aspect of the present invention. The dispenser comprises a body 400 in the form of a cassette holder which receives a cassette 402. The cassette has a mouthpiece 404 which is covered by a rotating lid 406 when the cassette 402 is *in situ* in the body 400.

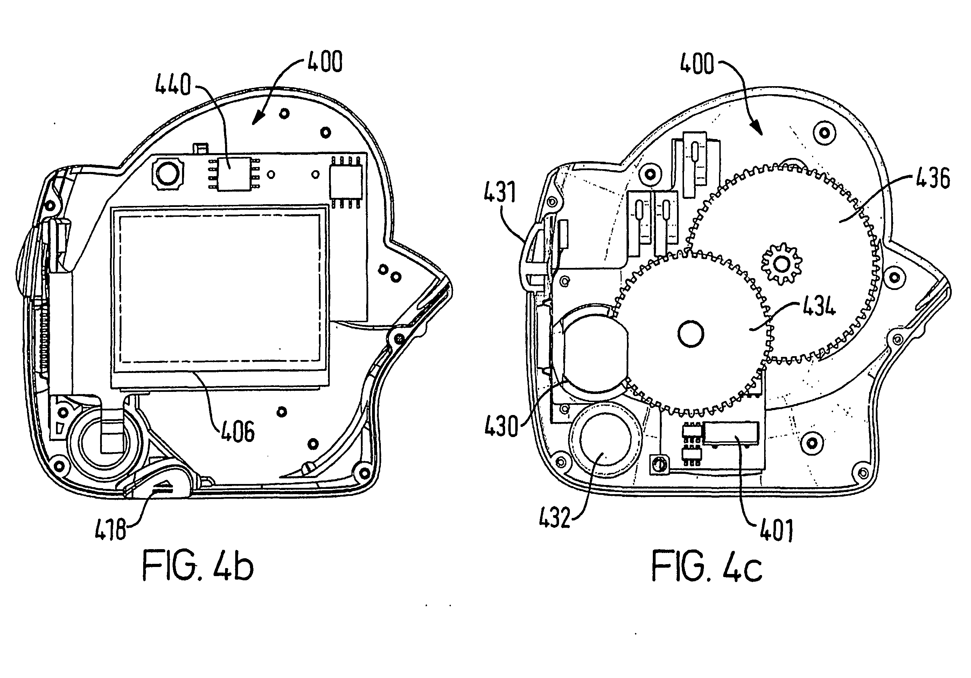

Figures 4b and 4c show a split-shell view of the holder 400 body shell. The body 400 is provided with DC motor 430 powered by battery 432, responsive to actuator switch 431. In use, the motor 430 drives gear wheels 434, 436, wherein gear wheel 436 drives foil spool 416 of the cassette 402 to advance a medicament dose. The body 400 is also provided with cassette release button 418 which releases a reversible catch mechanism (not shown) to enable mechanical release of a cassette 402 from the holder body 400. Electronic control system in the form of circuitry 440, 441 controls the DC motor 430, responsive to the actuator switch 431, and also controls LCD screen 406 for display of information to the user.

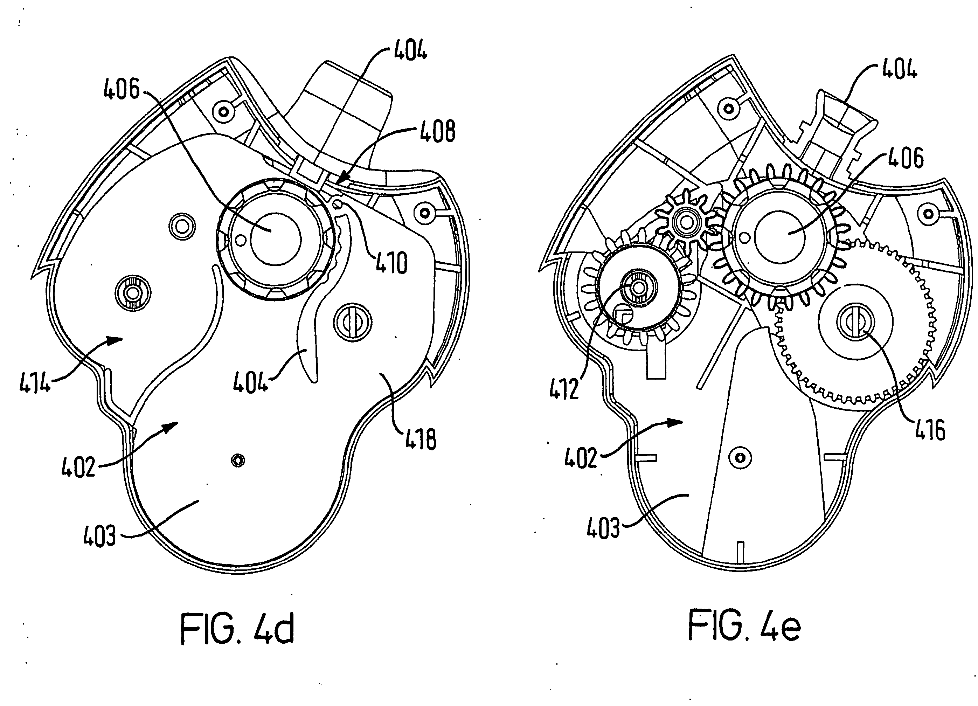

Figures 4d and 4e show the cassette 402 in more detail. A medicament strip (not shown for clarity) is positioned in chamber 403 of the cassette unit 402. The strip is pre-fed through a guide member 404 within the manifold component and engaged in a multi-pocket index wheel 406. The first pocket of the strip is positioned one pocket away from the opening station 408. The lid foil and base foil are separable about a beak 410. The resulting empty base foil is coiled about a base take-up spindle 412 in the base take-up chamber 414. The used lid foil is fed over the beak 410 and coiled about a lid take-up spindle 416 in the lid take-up chamber 418.

The dispenser of Figures 4a to 4e is actuated by pressing the actuator button 431 on the side of the dispenser which is linked to the electronic control system 440, 441 which controls DC motor 430 to index the internal mechanism by one pocket of medicament. The DC motor 430 thus indexes the strip and coils up the waste foils.

Initially, the gearing between the index wheel 406 and the lid take-up foil spindle 416 is one-to-one. However, as the lid take up spindle 416 winds on more foil, its effective winding diameter increases. An increase in diameter would cause the lid take-up spindle 416 to pull more strip than the index wheel 406 releases. Thus, in this particular example, the electronic control system 440, 441 is programmed to compensate for an increase in lid spool 416 diameter and adjust the amount the lid spool 416 rotates accordingly.

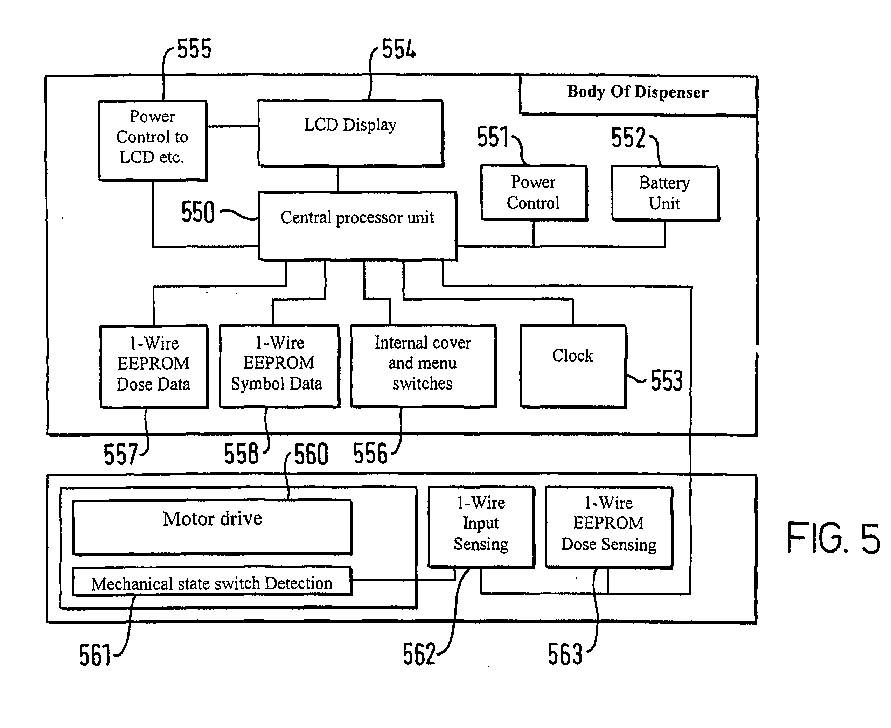

Figure 5 shows a schematic view of an electronic control system suitable for use in a medicament dispenser herein. The control system is typically located in the body/holder of a refillable medicament dispenser (e.g. as shown in Figures 4a to 4e). Central processor unit (CPU) 550 communicates with power control 551; battery 552; and dock 553. The CPU 550 also communicates with various user input/output functions which in combination comprise a man machine interface (MMI). In more detail, the MMI comprises an LCD display 554; power control for the LCD 555; and menu/switch inputs 556. Various memories including EEPROM dose data memory 557 and EEPROM symbol data memory 558 also communicate with the CPU 550.

Importantly, the control system 550 also communicates with the motor drive 560 and switch detector 561 therefor. Sensors are provided to detect user input 562 (e.g. manual actuation of the dispenser) and release of dose 563 from the dispenser. In embodiments, the dispenser is provided with a power management system enabling various power saving modes such as 'sleep' and 'powered down' modes.

It may be appreciated that any of the parts of the dispenser or cassette which contact the medicament suspension may be coated with materials such as fluoropolymer materials (e.g. PTFE or FEP) which reduce the tendency of medicament to adhere thereto. Any movable parts may also have coatings applied thereto which enhance their desired movement characteristics. Frictional coatings may therefore be applied to enhance frictional contact and lubricants (e.g. silicone oil) used to reduce frictional contact as necessary.

The medicament dispenser of the invention is suitable for dispensing medicament, particularly for the treatment of respiratory disorders such as asthma and chronic obstructive pulmonary disease (COPD), bronchitis and chest infections. Appropriate medicaments may thus be selected from, for example, analgesics, e.g., codeine, dihydromorphine, ergotamine, fentanyl or morphine; anginal preparations, e.g., diltiazem; antiallergics, e.g., cromoglycate (e.g. as the sodium salt), ketotifen or nedocromil (e.g. as the sodium salt); antiinfectives e.g., cephalosporins, penicillins, streptomycin, sulphonamides, tetracyclines and pentamidine; antihistamines, e.g., methapyrilene; anti- inflammatories, e.g., beclomethasone (e.g. as the dipropionate ester), fluticasone (e.g. as the propionate ester), flunisolide, budesonide, rofleponide, mometasone e.g. as the furoate ester), ciclesonide; triamcinolone (e.g. as the acetonide) or 6α, 9α-difluoro-11β-hydroxy-16α-methyl-3-oxo-17α-propionyloxy-androsta-1,4-diene-17β-carbothioic acid S-(2-oxo-tetrahydro-furan-3-yl) ester; antitussives, e.g., noscapine; bronchodilators, e.g., albuterol (e.g. as free base or sulphate), salmeterol (e.g. as xinafoate), ephedrine, adrenaline, fenoterol (e.g. as hydrobromide), formoterol (e.g. as fumarate), isoprenaline, metaproterenol, phenylephrine, phenylpropanolamine, pirbuterol (e.g. as acetate), reproterol (e.g. as hydrochloride), rimiterol, terbutaline (e.g. as sulphate), isoetharine, tulobuterol or 4-hydroxy-7-[2-[[2-[[3-(2-phenylethoxy)propyl]sulfonyl]ethyl]amino]ethyl-2(3H)-benzothiazolone; adenosine 2a agonists, e.g. 2R,3R,4S,5R)-2-[6-Amino-2-(1S-hydroxymethyl-2-phenyl-ethylamino)-purin-9-yl]-5-(2-ethyl-2H-tetrazol-5-yl)-tetrahydro-furan-3,4-diol (e.g. as maleate); α₄ integrin inhibitors e.g. (2S)-3-[4-({[4-(aminocarbonyl)-1-piperidinyl]carbonyl}oxy)phenyl]-2-[((2S)-4-methyl-2-{[2-(2-methylphenoxy) acetyl]amino}pentanoyl)amino] propanoic acid (e.g. as free acid or potassium salt), diuretics, e.g., amiloride; anticholinergics, e.g., ipratropium (e.g. as bromide), tiotropium, atropine or oxitropium; hormones, e.g., cortisone, hydrocortisone or prednisolone; xanthines, e.g., aminophylline, choline theophyllinate, lysine theophyllinate or theophylline; therapeutic proteins and peptides, e.g., insulin or glucagon; vaccines, diagnostics, and gene therapies. It will be clear to a person skilled in the art that, where appropriate, the medicaments may be used in the form of salts, (e.g., as alkali metal or amine salts or as acid addition salts) or as esters (e.g., lower alkyl esters) or as solvates (e.g., hydrates) to optimise the activity and/or stability of the medicament.

Preferred medicaments are selected from albuterol, salmeterol, fluticasone propionate and beclomethasone dipropionate and salts or solvates thereof, e.g., the sulphate of albuterol and the xinafoate of salmeterol.

Medicaments can also be delivered in combinations. Preferred formulations containing combinations of active ingredients contain salbutamol (e.g., as the free base or the sulphate salt) or salmeterol (e.g., as the xinafoate salt) or formoterol (eg as the fumarate salt) in combination with an anti-inflammatory steroid such as a beclomethasone ester (e.g., the dipropionate) or a fluticasone ester (e.g., the propionate) or budesonide. A particularly preferred combination is a combination of fluticasone propionate and salmeterol, or a salt thereof (particularly the xinafoate salt). A further combination of particular interest is budesonide and formoterol (e.g. as the fumarate salt).

Generally, powdered medicament particles suitable for delivery to the bronchial or alveolar region of the lung have an aerodynamic diameter of less than 10 micrometers, preferably less than 6 micrometers. Other sized particles may be used if delivery to other portions of the respiratory tract is desired, such as the nasal cavity, mouth or throat. The medicament may be delivered as pure drug, but more appropriately, it is preferred that medicaments are delivered together with excipients (carriers) which are suitable for inhalation. Suitable excipients include organic excipients such as polysaccharides (i.e. starch, cellulose and the like), lactose, glucose, mannitol, amino acids, and maltodextrins, and inorganic excipients such as calcium carbonate or sodium chloride. Lactose is a preferred excipient.

Particles of the powdered medicament and/or excipient may be produced by conventional techniques, for example by micronisation, milling or sieving. Additionally, medicament and/or excipient powders may be engineered with particular densities, size ranges, or characteristics. Particles may comprise active agents, surfactants, wall forming materials, or other components considered desirable by those of ordinary skill.

The excipient may be included with the medicament via well-known methods, such as by admixing, co-precipitating and the like. Blends of excipients and drugs are typically formulated to allow the precise metering and dispersion of the blend into doses. A standard blend, for example, contains 13000 micrograms lactose mixed with 50 micrograms drug, yielding an excipient to drug ratio of 260:1. Dosage blends with excipient to drug ratios of from 100:1 to 1:1 may be used. At very low ratios of excipient to drug, however, the drug dose reproducibility may become more variable.

It will be understood that the present disclosure is for the purpose of illustration only and the invention extends to modifications, variations and improvements thereto within the scope of the appended claims.

## Claims

1. A medicament dispenser (200) for use with a medicament carrier (100) having a plurality of pockets (104, 106, 108) for containing medicament wherein said pockets are spaced along the length of and defined between two peelable sheets (112, 114) secured to each other, said dispenser having an internal mechanism for accessing said medicament contained within said medicament carrier, said mechanism comprising,
a) an opening station (208) for receiving a pocket of said medicament carrier;
b) peeling means positioned to engage a base sheet (114) and a lid sheet (112) of a pocket which has been received in said opening station for peeling apart such a base sheet and lid sheet, to open such a pocket, said peeling means including lid driving (216) means for pulling apart a lid sheet and a base sheet of a pocket that has been received at said opening station;
c) an outlet (204), positioned to be in communication with an opened pocket through which a user can remove medicament from such an opened pocket; and
d) indexing means (206) for indexing in communication with said outlet, pockets of a medicament carrier in use with said medicament dispenser,
wherein said lid driving means comprises a wheel (216) on which the lid sheet is wound up, **characterised in that** said lid sheet wheel has an effective winding surface the diameter of which increases after every use of the dispenser as it winds on more lid sheet, and that said lid driving means are operated by an electronic drive system which comprises means to limit the extent of rotation of said wheel, in order to control the length of medicament carrier peeled by said peeling means so that the medicament carrier is indexed by the same amount on each use of the dispenser.

2. A medicament dispenser according to claim 1, wherein said electronic drive system is used in conjunction with a mechanical drive system.

3. A medicament dispenser (400) according to claim 1 or claim 2, wherein said electronic drive system comprises a DC electric motor (430).

4. A medicament dispenser (400) according to any one of the preceding claims further comprising actuating means (431) for actuating said electronic drive system.

5. A medicament dispenser according to claim 4, wherein said actuating means may take the form of a switch (431), push-button, or lever.

6. A medicament dispenser according to any one of the preceding claims, wherein said indexing means comprise a rotatable index wheel (206) having recesses therein, said index wheel being engageable with a medicament carrier in use with said medicament dispenser such that said recesses each receive a respective pocket of the base sheet of a medicament carrier in use with said medicament dispenser.

7. A medicament dispenser (300) according to any one of the preceding claims, wherein the internal mechanism for accessing the said medicament contained within said medicament carrier is housed within a cassette (304).

8. A medicament dispenser (300) according to claim 7 comprising,
a body (300);
a holder, (302) shaped to fit within said body and movable relative to said body; and
receivable by said holder, said cassette containing said medicament carrier.

9. A medicament dispenser according to claim 8, wherein movement of said holder relative to said body results in movement of said cassette between a first position and a second position such that the cassette is reversibly removable from the holder when the cassette is in the second position.

10. A medicament dispenser according to claim 8 or claim 9, wherein the cassette additionally comprises a mouthpiece.

11. A medicament dispenser according to any one of the preceding claims wherein the medicament is in powdered form.

12. A medicament dispenser according to claim 11, wherein the medicament comprises a drug selected from the group consisting of albuterol, salmeterol, fluticasone propionate and beclomethasone dipropionate and salts or solvates thereof and any combination thereof.

13. A medicament dispenser according to claim 12, wherein said combination comprises salmeterol xinafoate and fluticasone propionate.

14. A medicament dispenser according to any one of the preceding claims including the medicament carrier.

## Patentansprüche

1. Medikamentenspender (200) zur Verwendung mit einem Medikamententräger (100) mit einer Vielzahl von Taschen (104, 106, 108) zur Aufnahme eines Medikaments, wobei die Taschen entlang der Länge von zwei ablösbaren Bögen (112, 114), die aneinander befestigt sind, beabstandet sind, und sie zwischen den Bögen definiert werden, wobei der Spender einen inneren Mechanismus zum Zugriff auf das innerhalb des Medikamententrägers enthaltene Medikament aufweist, und der Mechanismus aufweist:
a) eine Öffnungsstelle (208) zum Empfangen einer Tasche des Medikamententrägers;
b) eine Ablöse-Einrichtung, die positioniert ist, um einen Grundbogen (114) und einen Deckbogen (112) von einer Tasche in Eingriff zu nehmen, die in der Öffnungsstelle zum Lösen eines derartigen Grundbogens und Deckbogens voneinander empfangen wurde, um eine derartige Tasche zu öffnen, wobei die Ablöse-Einrichtung eine Deckelantriebseinrichtung (216) aufweist, zum Auseinanderziehen eines Deckbogens und eines Grundbogens einer Tasche, die an der Öffnungsstelle empfangen wurde;
c) einen Auslass (204), der positioniert ist, um sich mit einer geöffneten Tasche in Verbindung zu befinden, durch die ein Nutzer ein Medikament aus einer derartigen geöffneten Tasche entnehmen kann; und
d) eine Indizierungseinrichtung (206) zum Indizieren, in Verbindung mit dem Auslass, der Taschen eines mit dem Medikamentenspender verwendeten Medikamententrägers;
wobei die Deckelantriebseinrichtung ein Rad (216) aufweist, an dem der Deckbogen aufgewickelt wird;
**dadurch gekennzeichnet, dass** das Deckbogen-Rad eine effektive Wicklungsoberfläche aufweist, deren Durchmesser nach jeder Nutzung des Spenders zunimmt, da es mehr Deckbogen aufwickelt, und dass die Deckelantriebseinrichtung durch ein elektronisches Antriebssystem betrieben wird, das eine Einrichtung aufweist, um das Ausmaß der Drehung des Rads zu begrenzen, um die Länge des Medikamententrägers zu steuern, die durch die Ablöse-Einrichtung gelöst wird, so dass der Medikamententräger durch die gleiche Menge bei jeder Verwendung des Spenders indiziert wird.

2. Medikamentenspender nach Anspruch 1, bei dem das elektronische Antriebssystem in Verbindung mit einem mechanischen Antriebssystem verwendet wird.

3. Medikamentenspender (400) nach Anspruch 1 oder Anspruch 2, bei dem das elektronische Antriebssystem einen elektrischen DC-Motor (430) aufweist.

4. Medikamentenspender (400) nach einem der vorhergehenden Ansprüche, ferner mit einer Betätigungseinrichtung (431) zum Betätigen des elektronischen Antriebssystems.

5. Medikamentenspender nach Anspruch 4, bei dem die Betätigungseinrichtung die Form eines Schalters (431), einer Drucktaste oder eines Hebels annehmen kann.

6. Medikamentenspender nach einem der vorhergehenden Ansprüche, bei dem die Indizierungseinrichtung ein drehbares Index-Rad (206) mit Aussparungen darin aufweist, wobei das Index-Rad mit einem Medikamententräger, der mit dem Medikamentenspender verwendet wird, in Eingriff bringbar ist, derart, dass jede der Aussparungen eine jeweilige Tasche des Grundbogens eines mit dem Medikamentenspender verwendeten Medikamententrägers empfängt.

7. Medikamentenspender (300) nach einem der vorhergehenden Ansprüche, bei dem der innere Mechanismus zum Zugriff auf das innerhalb des Medikamententrägers enthaltene Medikament innerhalb einer Kassette (304) aufgenommen wird.

8. Medikamentenspender (300) nach Anspruch 7, mit:
einem Körper (300);
einem Halter (302), der derart geformt ist, dass er in den Körper passt und relativ zu dem Körper bewegbar ist; und
durch den Halter empfangbar ist, wobei die Kassette den Medikamententräger enthält.

9. Medikamentenspender nach Anspruch 8, bei dem die Bewegung des Halters relativ zu dem Körper in einer Bewegung der Kassette zwischen einer ersten Position und einer zweiten Position resultiert, derart, dass die Kassette von dem Halter reversibel entfernbar ist, wenn sich die Kassette in der zweiten Position befindet.

10. Medikamentenspender nach Anspruch 8 oder Anspruch 9, bei dem die Kassette zusätzlich ein Mundstück aufweist.

11. Medikamentenspender nach einem der vorhergehenden Ansprüche, bei dem das Medikament in Pulverform vorliegt.

12. Medikamentenspender nach Anspruch 11, bei dem das Medikament ein Arzneimittel aufweist, das aus der Gruppe ausgewählt wird, die aus Albuterol, Salmeterol, Fluticason-Propionat und Beclomethason-Dipropionat und Salzen oder Solvaten davon, und jeglichen Kombination davon, besteht.

13. Medikamentenspender nach Anspruch 12, bei dem die Kombination Salmeterol-Xinafoat und Fluticason-Propionat aufweist.

14. Medikamentenspender nach einem der vorhergehenden Ansprüche, den Medikamententräger enthaltend.

## Revendications

1. Distributeur de médicament (200) pour utilisation avec un transporteur de médicament (100) présentant un certain nombre de poches (104, 106, 108) destinées à contenir un médicament, dans lequel lesdites poches sont espacées sur la longueur de et définies entre deux feuilles détachables (112, 114) fixées l'une à l'autres, ledit distributeur ayant un mécanisme intérieur pour accéder audit médicament contenu dans ledit transporteur de médicament, ledit mécanisme comprenant :
a) une station d'ouverture (208) pour recevoir une poche dudit transporteur de médicament ;
b) un moyen de décollement positionné de façon à engager une feuille de base (114) et une feuille de recouvrement (112) de la poche qui a été reçue dans ladite station d'ouverture pour détacher ces feuilles de base et de recouvrement, pour ouvrir cette poche, ledit moyen de décollement comprenant un moyen d'entraînement du recouvrement (216) pour tirer séparément une feuille de base et une feuille de recouvrement d'une poche qui a été reçue à ladite station d'ouverture ;
c) une sortie (204) disposée de façon à être en communication avec une poche ouverte à travers laquelle un utilisateur peut retirer un médicament de cette poche ouverte : et
d) un moyen d'indexation (206) pour indexer, en communication avec ladite ouverture, les poches d'un transporteur de médicament utilisé avec ledit distributeur de médicament,
dans lequel ledit moyen d'entraînement de recouvrement comprend une roue (216) sur laquelle la feuille de recouvrement est enroulée, **caractérisé en ce que** ladite roue de feuille de recouvrement présente une surface d'enroulement effectif dont le diamètre augmente après chaque utilisation du distributeur à mesure qu'elle enroule davantage de feuille de recouvrement et **en ce que** ledit moyen d'entraînement de recouvrement est mis en oeuvre par un système électronique d'entraînement qui comprend un moyen pour limiter l'accroissement de rotation de ladite roue, dans le but de contrôler la longueur du transporteur de médicament décollée par ledit moyen de décollement, de sorte que le transporteur de médicament soit indexé de la même quantité à chaque utilisation du distributeur.

2. Distributeur de médicament selon la revendication 1, dans lequel ledit système électronique d'entraînement est employé conjointement avec un système mécanique d'entraînement.

3. Distributeur de médicament selon la revendication 1 ou la revendication 2, dans lequel ledit système électronique d'entraînement (400) comprend un moteur électrique à courant continu (430).

4. Distributeur de médicament selon l'une quelconque des revendications précédentes comprenant en outre un moyen d'actionnement (431) pour actionner ledit système électronique d'entraînement.

5. Distributeur de médicament selon la revendication 4, dans lequel ledit moyen d'actionnement peut prendre la forme d'un interrupteur (431), d'un bouton poussoir, ou d'un levier.

6. Distributeur de médicament selon l'une quelconque des revendications précédentes, dans lequel ledit système d'indexation comprend une roue d'indexation tournante (206) comportant des encoches, ladite roue d'indexation pouvant s'engager avec un transporteur de médicament utilisé avec ledit distributeur de médicament de façon à ce que lesdites encoches reçoivent chacune une poche respective de la feuille de base d'un transporteur de médicament employé avec ledit distributeur de médicament.

7. Distributeur de médicament (300) selon l'une quelconque des revendications précédentes revendication 1, dans lequel le mécanisme intérieur d'accession audit médicament contenu dans ledit transporteur de médicament est logé dans une cassette (304).

8. Distributeur de médicament (300) selon la revendication 7, comprenant :
un corps (300) ;
un support (302), dimensionné pour s'ajuster dans ledit corps et mobile par rapport audit corps ; et pouvant être reçue par ledit support, ladite cassette contenant ledit transporteur de médicament.

9. Distributeur de médicament selon la revendication 8, dans lequel le déplacement dudit support par rapport audit corps a pour effet le déplacement de ladite cassette entre une première position et une seconde position de telle sorte que la cassette puisse être retirée du support de façon réversible lorsque la cassette est dans la seconde position.

10. Distributeur de médicament selon la revendication 8 ou la revendication 9, dans lequel la cassette comprend en outre un embout.

11. Distributeur de médicament selon l'une quelconque des revendications précédentes, dans lequel le médicament est sous forme de poudre.

12. Distributeur de médicament selon la revendication 11, dans lequel le médicament comprend un médicament choisie dans le groupe composé de l'albutérol, le salmétérol, le propionate de fluticasone et le dipropionate de béclométhasone et des sels ou des solvats de ceux-ci et toute combinaison de ceux-ci.

13. Distributeur de médicament selon la revendication 12, dans lequel ladite combinaison comprend le xinafoate de salmétérol et le propionate de fluticasone.

14. Distributeur de médicament selon l'une quelconque des revendications précédentes comportant le transporteur de médicament.
